# EUROPÄISCHE PATENTSCHRIFT

(11) **EP 3 295 896 B1**
(45) Veröffentlichungstag und Bekanntmachung des Hinweises auf die Patenterteilung: **16.12.2020**
(21) Anmeldenummer: 16189432.4
(22) Anmeldetag: 19.09.2016
(51) Int. Cl.: A61C 19/00, A61B 90/70

(54) **MEDIZINISCHE ODER DENTALE REINIGUNGS- UND / ODER PFLEGEVORRICHTUNG**
MEDICAL OR DENTAL CLEANING- AND / OR MAINTENANCE-DEVICE
DISPOSITIF DE SOIN ET/OU DE NETTOYAGE DENTAIRE OU MEDICAL

(43) Veröffentlichungstag der Anmeldung: 21.03.2018
(73) Patentinhaber: W & H Dentalwerk Bürmoos GmbH, 5111 Bürmoos (AT)
(72) Erfinder: GALLUSEDER, Florian, 5121 Tarsdorf (AT); MEBURGER, Jürgen, 5112 Lamprechtshausen (AT); EIBL, Johann, 5230 Mattighofen (AT)
(74) Vertreter: Benda, Ralf

(56) Entgegenhaltungen:
- EP-A1- 2 364 665
- DE-A1-102014 103 071
- US-A1- 2013 098 407

## Beschreibung

Die vorliegende Erfindung betrifft eine medizinische oder dentale Reinigungs- und / oder Pflegevorrichtung zur Reinigung und / oder Pflege zumindest eines medizinischen oder dentalen Instruments.

Aus der Patentanmeldung EP 2 364 665 A1 ist eine medizinische oder dentale Reinigungs- und / oder Pflegevorrichtung mit einer Reinigungskammer bekannt, in die mehrere zu reinigende oder pflegende Instrumente einbringbar sind. Jedes Instrument wird zur Versorgung mit einem Reinigungs- oder Pflegemittel mit einem Anschluss an eine Versorgungsvorrichtung verbunden, wobei die Anschlüsse auf einem Basiselement angeordnet sind. Das Basiselement ist drehbar in der Kammer angeordnet, so dass während eines Reinigungs- und / oder Pflegeprozesses jeweils ein Anschluss mit dem daran befestigten Instrument mit der Versorgungsvorrichtung verbunden ist, während zumindest ein weiterer Anschluss nicht mit der Versorgungsvorrichtung verbunden ist. Mit dieser bekannten Reinigungs- und / oder Pflegevorrichtung ist somit eine sequentielle Reinigung und / oder Pflege mehrere in die Reinigungskammer eingebrachter Instrumente möglich. Wie beschrieben werden dabei zuerst alle zu reinigenden und / oder zu pflegenden Instrumente in die Reinigungskammer eingebracht, anschließend die Instrumente der Reihe nach gereinigt und / oder gepflegt und nach Abschluss der Reinigung und / oder Pflege aller in der Reinigungskammer aufgenommenen Instrumente diese aus der Reinigungskammer entfernt.

Der vorliegenden Erfindung liegt die Aufgabe zugrunde eine alternative medizinische oder dentale Reinigungs- und / oder Pflegevorrichtung zu schaffen, deren Handhabung für den Anwender einfacher und zeitsparender ist.

Diese Aufgabe wird gemäß der vorliegenden Erfindung durch eine medizinische oder dentale Reinigungs- und / oder Pflegevorrichtung zur Reinigung und / oder Pflege zumindest eines medizinischen oder dentalen Instruments mit den Merkmalen des Anspruchs 1 und durch ein Verfahren zum Betrieb einer medizinischen oder dentalen Reinigungs- und / oder Pflegevorrichtung nach Anspruch 13 gelöst. Besonders vorteilhafte Ausführungsformen sind in den jeweiligen Unteransprüchen angeführt.

Die erfindungsgemäße medizinische oder dentale Reinigungs- und / oder Pflegevorrichtung zur Reinigung und / oder Pflege zumindest eines medizinischen oder dentalen Instruments, umfasst: ein Gehäuse, mehrere Kammern zur Aufnahme von zumindest jeweils einem zu reinigenden und / oder zu pflegenden medizinischen oder dentalen Instrument, wobei jede Kammer zumindest ein Abgabeelement zur Abgabe zumindest eines Reinigungs- und / oder Pflegemittels in die jeweilige Kammer und / oder an das zumindest eine, in der jeweiligen Kammer aufgenommene, zu reinigende und / oder zu pflegende medizinische oder dentale Instrument aufweist, eine (einzige oder gemeinsame) Öffnung in dem Gehäuse, durch welche die mehreren Kammern mit medizinischen oder dentalen Instrumenten beladen werden können, und eine Abdeckung zum Verschließen der Öffnung und der mehreren Kammern, insbesondere während eines Reinigungs- und / oder Pflegevorgangs. Durch die Abdeckung ist nur ein Teil der Öffnung verschließbar oder abdeckbar, so dass, wenn zumindest eine (erste) Kammer der mehreren Kammern vollständig verschlossen ist, und insbesondere mit einem Reinigungs- und / oder Pflegemittel versorgt wird oder werden kann, zumindest eine andere oder zweite Kammer der mehreren Kammern nicht (vollständig) verschlossen ist und / oder durch die Öffnung mit einem medizinischen oder dentalen Instrument beladbar ist und somit insbesondere nicht mit einem Reinigungs- und / oder Pflegemittel versorgt wird.

Es ist somit zumindest eine (erste) Kammer der mehreren Kammern durch die Abdeckung vollständig verschließbar oder verschlossen, während eine andere oder zweite Kammer nicht vollständig verschließbar oder abdeckbar ist.

Durch die Abdeckung ist nur ein Teil der Öffnung verschließbar, so dass, zusätzlich oder alternativ zu dem Vorstehenden, die Reinigungs- und / oder Pflegevorrichtung, insbesondere eine im Nachstehenden noch detaillierter beschriebene Steuer- oder Regelvorrichtung, ausgebildet ist, ein Reinigungs- und / oder Pflegemittel nur an oder in eine Kammer der mehreren Kammern zu fördern, die vollständig verschlossen ist, während eine andere Kammer, die nicht vollständig verschlossen ist, nicht mit einem Reinigungs- und / oder Pflegemittel versorgt wird.

Es ist somit eine sequentiell und / oder alternierend betreibbare oder beladbare medizinische oder dentale Reinigungs- und / oder Pflegevorrichtung zur Reinigung und / oder Pflege zumindest eines medizinischen oder dentalen Instruments geschaffen, bei der in vorteilhafter Weise ein kontinuierliches Arbeiten möglich ist: Während in zumindest einer (vollständig verschlossenen) Kammer ein medizinisches oder dentales Instrument mit einem Reinigungs- und / oder Pflegemittel versorgt oder gereinigt und / gepflegt wird, kann ein Anwender ein bereits gereinigtes und / oder gepflegtes Instrument aus einer anderen (zugänglichen oder nicht vollständig verschlossenen) Kammer entnehmen und diese Kammer dann mit einem weiteren, zu reinigenden und / oder zu pflegenden Instrument beladen.

Vorzugsweise ist die medizinische oder dentale Reinigungs- und / oder Pflegevorrichtung ausgebildet, ein medizinisches oder dentales Instrument, insbesondere dessen Innenraum und / oder in dem Innenraum angeordnete Bauteil, über die Abgabeelemente mit zumindest einem Reinigungs- und / oder Desinfektions- und / oder Schmiermittel zu versorgen. Vorzugsweise ist die medizinische oder dentale Reinigungs- und / oder Pflegevorrichtung ausgebildet, ein in dem medizinischen oder dentalen Instrument vorgesehenes Element, zum Beispiel eine Leitung, einen Kanal oder ein Rohr für den Transport von Gas, Flüssigkeiten, Luft oder Wasser, mit einem Reinigungs- und / oder Desinfektionsmittel zu reinigen oder durchzuspülen. Vorzugsweise ist die medizinische oder dentale Reinigungs- und / oder Pflegevorrichtung ausgebildet, bewegliche Teile eines medizinischen oder dentalen Instruments, insbesondere Lagerelemente, zum Beispiel Kugellager, mit einem Schmiermittel zu versorgen.

Vorzugsweise ist die medizinische oder dentale Reinigungs- und / oder Pflegevorrichtung ausgebildet, ein medizinisches oder dentales Instrument in einem Reinigungs- und / oder Pflegeprozess von kurzer Dauer zu reinigen und / oder zu pflegen. Der Reinigungs- und / oder Pflegeprozess dauert zum Beispiel weniger als 60 Sekunden, vorzugsweise weniger als 30 Sekunden, insbesondere weniger als 20 Sekunden, besonders bevorzugt in etwa 10 Sekunden.

Vorzugsweise umfasst die medizinische oder dentale Reinigungs- und / oder Pflegevorrichtung eine Versorgungsvorrichtung, die ausgebildet ist, die Abgabeelemente der Kammern mit zumindest einem Reinigungs- und / oder Pflegemittel zu versorgen. Vorzugsweise verbindet die Versorgungsvorrichtung die Abgabeelemente mit Quellen für die Reinigungs- und / oder Pflegemittel, zum Beispiel mit Behältern für die Reinigungs- und / oder Pflegemittel. Vorzugsweise umfasst die Versorgungsvorrichtung Leitungen, Rohre und / oder Kanäle zum Verbinden der Reinigungs- und / oder Pflegemittelquellen mit den Abgabeelementen. Vorzugsweise umfasst die Versorgungsvorrichtung zumindest ein Steuerelement, zum Beispiel zumindest ein Ventil, eine Drossel oder eine Pumpe, zum gesteuerten und / oder geregelten Fördern eines Reinigungs- und / oder Pflegemittels. Vorzugsweise umfasst die Versorgungsvorrichtung zumindest ein Detektor- oder Sensorelement, das ausgebildet ist, zumindest eine Stellgröße oder einen Zustand eines Elements der Versorgungsvorrichtung, zum Beispiel die Öffnungsposition eines Ventils, die Fördermenge einer Pumpe oder die Füllmenge eines Reinigungs- und / oder Pflegemittels in einem Behälter, zu erkennen. Vorzugsweise umfasst die Versorgungsvorrichtung zumindest ein Detektor- oder Sensorelement, das ausgebildet ist, einen Parameter eines Reinigungs- und / oder Pflegemittels, zum Beispiel dessen Durchfluss- oder Fördermenge, Druck oder Leitfähigkeit, zu bestimmen.

Vorzugsweise ist die Versorgungsvorrichtung mit einer Steuer- oder Regelvorrichtung der medizinischen oder dentalen Reinigungs- und / oder Pflegevorrichtung verbunden. Vorzugsweise umfasst die Steuer- oder Regelvorrichtung einen Mikrokontroller. Bevorzugt ist die Steuer- oder Regelvorrichtung ausgebildet, die Versorgungsvorrichtung und somit vorzugsweise die Förderung, insbesondere die sequentielle und / oder alternierende Förderung, des zumindest einen Reinigungs- und / oder Pflegemittels in die Kammern und / oder an die Abgabeelemente zu steuern und / oder zu regeln. Vorzugsweise ist die Steuer- oder Regelvorrichtung ausgebildet, ein Signal des zumindest einen Detektor- oder Sensorelements der Versorgungsvorrichtung zu empfangen und zu verarbeiten und auf Basis dieses Signals zumindest ein Element, insbesondere eines der vorstehenden Steuerelemente, der Versorgungsvorrichtung zu betreiben. Besonders bevorzugt ist die Steuer- oder Regelvorrichtung operativ auch mit einer im Nachstehenden noch im Detail beschriebenen Detektoreinheit verbunden, die ausgebildet ist, zumindest eine Position der Abdeckung zu erkennen.

Vorzugsweise ist zumindest eine Teil der Versorgungsvorrichtung und / oder die Steuer- oder Regelvorrichtung in dem Gehäuse der medizinischen oder dentalen Reinigungs- und / oder Pflegevorrichtung angeordnet.

Vorzugsweise umfasst die medizinische oder dentale Reinigungs- und / oder Pflegevorrichtung zwei Kammern. Alternativ ist es jedoch auch denkbar, dass die medizinische oder dentale Reinigungs- und / oder Pflegevorrichtung mehr als zwei Kammern, zum Beispiel drei, vier, fünf oder n Kammern aufweist. Vorzugsweise ist jede Kammer ausgebildet oder dazu vorgesehen, ein (einziges) zu reinigendes und / oder zu pflegendes Instrument aufzunehmen. Alternativ ist es jedoch auch denkbar, dass zumindest eine Kammer zur Aufnahme von mehr als einem zu reinigenden und / oder zu pflegenden Instrument ausgebildet oder vorgesehen ist.

Vorzugsweise umfasst jede Kammer ein (einziges) Abgabeelement zur Abgabe zumindest eines Reinigungs- und / oder Pflegemittels, an das insbesondere ein oder das pro Kammer einzige zu reinigende und / oder zu pflegende Instrument anschließbar ist. Alternativ ist es jedoch auch denkbar, dass eine Kammer mehr als ein Abgabeelement aufweist, wobei an jedes Abgabeelement einer Kammer ein zu reinigendes und / oder zu pflegendes Instrument anschließbar ist.

Vorzugsweise weist das Abgabeelement zumindest eine Bohrung, Leitung oder einen Kanal auf, durch die / den ein Reinigungs- und / oder Pflegemittel in eine der Kammern und / oder an ein in einer Kammer aufgenommenes zu reinigendes und / oder zu pflegendes Instrument förderbar ist. Vorzugsweise ist das Abgabeelement ausgebildet, in eine Aufnahme oder ein Kupplungselement eines zu reinigenden und / oder zu pflegenden Instruments einführbar zu sein, so dass das Reinigungs- und / oder Pflegemittel in das Innere des Instruments förderbar ist. Bevorzugt ist das Abgabeelement als rohrförmiger Stutzen ausgebildet, der insbesondere in eine Aufnahme oder ein Kupplungselement eines zu reinigenden und / oder zu pflegenden Instruments einführbar ist. Besonders bevorzugt ist die zumindest eine Bohrung, Leitung oder der Kanal des Abgabeelements derart angeordnet, dass sie / er mit einer in dem medizinischen oder dentalen Instrument vorgesehenen Leitung oder Kanal oder einem Rohr verbindbar ist, so dass ein Reinigungs- und / oder Pflegemittel in die Leitung, den Kanal oder das Rohr des medizinischen oder dentalen Instruments förderbar ist.

Alternativ ist es auch denkbar, dass das Abgabeelement ausgebildet ist, ein Reinigungs- und / oder Pflegemittel an eine Außenseite eines zu reinigenden und / oder zu pflegenden Instruments abzugeben. Vorzugsweise umfasst ein derartiges Abgabeelement eine Düse.

Gemäß einem Ausführungsbeispiel umfasst eine medizinische oder dentale Reinigungs- und / oder Pflegevorrichtung eine erste Kammer mit zumindest einem Abgabeelement zur Abgabe eines Reinigungs- und / oder Pflegemittels an eine Außenseite eines zu reinigenden und / oder zu pflegenden Instruments und eine zweite (von der ersten Kammer getrennte) Kammer mit zumindest einem Abgabeelement zum Einführen in eine Aufnahme oder ein Kupplungselement eines zu reinigenden und / oder zu pflegenden Instruments. Alternativ ist es auch denkbar, dass eine (einzige) Kammer der medizinischen oder dentalen Reinigungs- und / oder Pflegevorrichtung zumindest ein Abgabeelement zur Abgabe eines Reinigungs- und / oder Pflegemittels an eine Außenseite eines zu reinigenden und / oder zu pflegenden Instruments und zumindest ein Abgabeelement zum Einführen in eine Aufnahme oder ein Kupplungselement eines zu reinigenden und / oder zu pflegenden Instruments aufweist.

Die Öffnung in dem Gehäuse der medizinischen oder dentalen Reinigungs- und / oder Pflegevorrichtung, durch welche die mehreren Kammern mit medizinischen oder dentalen Instrumenten beladen werden können, ist insbesondere als einzige oder gemeinsame Öffnung ausgebildet, so dass insbesondere alle Kammern der Reinigungs- und / oder Pflegevorrichtung durch diese Öffnung zugänglich sind, d.h. mit jeweils zumindest einem Instrument beladbar sind und / oder das zumindest eine Instrument daraus entnehmbar ist. Die Öffnung erstreckt sich vorzugsweise über alle Kammern der dentalen Reinigungs- und / oder Pflegevorrichtung. Die Öffnung ist vorzugsweise in einer Frontseite des Gehäuses der Reinigungs- und / oder Pflegevorrichtung vorgesehen. Die Öffnung und / oder ein die Öffnung begrenzender, zum Beispiel durch das Gehäuse gebildeter Rand ist / sind vorzugsweise viereckig geformt.

Die Abdeckung zum Verschließen der Öffnung und der mehreren Kammern während eines Reinigungs- und / oder Pflegevorgangs ist vorzugsweise als Tür oder Deckel ausgebildet. Die Abdeckung, insbesondere die Tür oder der Deckel, ist vorzugsweise einteilig und / oder viereckig, insbesondere rechteckig, ausgebildet.

Wie im Vorstehenden bereits erwähnt ist die Abdeckung vorzugsweise derart bemessen, dass zumindest eine Kammer der mehreren Kammern durch die Abdeckung vollständig verschließbar oder abdeckbar ist, während eine andere oder zweite Kammer nicht vollständig verschließbar oder abdeckbar ist. Anders ausgedrückt ist die Abdeckung vorzugsweise derart bemessen, dass sie nicht alle Kammern der Reinigungs- und / oder Pflegevorrichtung gleichzeitig verschließt oder abdeckt. Vorzugsweise ist die Fläche der Abdeckung, insbesondere der Außenseite oder äußere Oberfläche der Abdeckung, kleiner als die Fläche der Öffnung durch welche die mehreren Kammern mit medizinischen oder dentalen Instrumenten beladen werden können bzw. ist die Fläche der Öffnung größer als die Fläche der Abdeckung. Besonders bevorzugt umfasst die medizinische oder dentale Reinigungs- und / oder Pflegevorrichtung zwei Kammern, wobei das Verhältnis der Fläche der Abdeckung zur Fläche der Öffnung in etwa 1:2 beträgt.

Vorzugsweise sind die mehreren Kammern durch jeweils zumindest ein, insbesondere festes oder starres, Wandelement voneinander getrennt. Vorzugsweise ist das zumindest eine Wandelelement mit dem Gehäuse der Reinigungs- und / oder Pflegevorrichtung verbunden oder als Teil davon ausgebildet. Vorzugsweise trennt das zumindest eine Wandelement zwei benachbarte Kammern, insbesondere gemeinsam mit der Abdeckung, derart voneinander, dass kein Reinigungs- und / oder Pflegemittel von einer Kammer in eine andere Kammer übertritt. Vorzugsweise ist das zumindest eine Wandelement zwischen zwei Abgabeelementen angeordnet oder trennt diese voneinander, so dass insbesondere in jeder Kammer nur ein Abgabeelement angeordnet ist. Vorzugsweise ist das zumindest eine Wandelement im Wesentlichen rechtwinkelig zu einem Kammerboden einer Kammer angeordnet. Vorzugsweise ist an dem Wandelement, insbesondere an einem Verbindungsbereich zwischen dem Wandelement und dem Kammerboden, eine Rinne zum Ableiten von Reinigungs- und / oder Pflegemittel vorgesehen. Das zumindest eine Wandelement ermöglich damit neben einem sequentiellen und / oder alternierenden Betrieb auch eine saubere Funktion der Reinigungs- und / oder Pflegevorrichtung, in dem es ein Übertragen von Reinigungs- und / oder Pflegemittel zwischen den mehreren Kammern verhindert.

Vorzugsweise ist die Abdeckung beweglich, insbesondere verschiebbar, in oder an der Öffnung angeordnet. Alternativ oder zusätzlich ist die Abdeckung in oder entlang der Öffnung bewegbar, insbesondere verschiebbar. Vorzugsweise ist die Abdeckung geradlinig bewegbar oder verschiebbar, insbesondere entlang oder parallel zu einem Rand der Öffnung oder einer Gehäusekante, welche die Öffnung begrenzt. Damit ist eine einfach herzustellende und unkompliziert zu bedienende Abdeckvorrichtung geschaffen.

Vorzugsweise ist die Abdeckung in eine Abdeck- oder Verschlussposition bewegbar, insbesondere verschiebbar, in der die Abdeckung zumindest eine der mehreren Kammern (vollständig) verschließt oder abdeckt. Vorzugsweise ist die Abdeckung in mehrere (separate oder diskrete) Abdeck- oder Verschlussposition bewegbar, insbesondere verschiebbar, so dass jede der mehreren Kammern (vollständig) durch die Abdeckung verschließbar oder abdeckbar ist. Vorzugsweise ist die Abdeckung derart ausgebildet oder bemessen, dass sie jeweils nur eine der mehreren Abdeck- oder Verschlussposition einnimmt und / oder jeweils nur eine der mehreren Kammern (vollständig) verschließt oder abdeckt und / oder zumindest eine der mehreren Kammern nicht (vollständig) verschließt oder abdeckt. Vorzugsweise ist die Abdeckung in zumindest eine Verschiebe- oder Zwischenposition bewegbar, insbesondere verschiebbar, in der sie keine der mehreren Kammern vollständig verschließt oder abdeckt. Vorzugsweise ist die Abdeckung derart ausgebildet oder bemessen, dass sie, um von einer ersten Abdeck- oder Verschlussposition, in der sie eine erste der mehreren Kammern (vollständig) verschließt oder abdeckt, in eine zweite Abdeck- oder Verschlussposition, in der sie eine andere, zweite der mehreren Kammern (vollständig) verschließt oder abdeckt, zu gelangen, zumindest eine Verschiebe- oder Zwischenposition einnimmt.

Vorzugsweise ist die Öffnung durch einen (durch das Gehäuse gebildeten) Rand oder eine Gehäusekante begrenzt, an dem bevorzugt zumindest ein Führungselement zum Bewegen, insbesondere zum Verschieben, der Abdeckung vorgesehen ist. Das Führungselement umfasst zum Beispiel eine Leiste oder eine Nut. Vorzugsweise ist an der Abdeckung ein an das Führungselement abgestimmtes Führungsteil vorgesehen, zum Beispiel eine Leiste oder eine Nut, das mit dem Führungselement derart verbindbar ist, dass die Abdeckung in oder an der Öffnung bewegbar, insbesondere verschiebbar, ist. Das Führungselement und das Führungsteil greifen insbesondere ineinander.

Alternativ oder zusätzlich ist der Rand des Gehäuses oder die Gehäusekante ausgebildet, die Bewegung, insbesondere das Verschieben, der Abdeckung zu begrenzen. Der Rand oder die Gehäusekante ist insbesondere als Anschlag oder Stopp für die Abdeckung ausgebildet. Insbesondere ist vorgesehen, dass eine Seitenkante der Abdeckung und der Rand oder die Gehäusekante einander kontaktieren, um die Bewegung, insbesondere das Verschieben, der Abdeckung zu begrenzen.

Besonders bevorzugt weist der Rand oder die Gehäusekante mehrere gewinkelt zueinander angeordnete Seiten auf, wobei an zumindest einer (ersten) Seite ein Führungselement zum Bewegen, insbesondere zum Verschieben, der Abdeckung vorgesehen ist und eine andere (zweite) Seite als Anschlag oder Stopp für die Abdeckung ausgebildet ist. Besonders bevorzugt weist die Abdeckung mehrere gewinkelt zueinander angeordnete Seiten auf, wobei an zumindest einer (ersten) Seite ein Führungsteil zum Bewegen, insbesondere zum Verschieben, der Abdeckung vorgesehen ist und eine andere (zweite) Seite als Seitenkante dient, die den Anschlag oder Stopp kontaktiert. Durch diese im Vorstehenden genannten Merkmale ist eine einfach herzustellende und unkompliziert zu bedienende Abdeckvorrichtung geschaffen.

Alternativ oder zusätzlich ist es auch möglich, dass an dem zumindest einen Wandelement zumindest ein Führungselement zum Bewegen, insbesondere zum Verschieben, der Abdeckung vorgesehen ist.

Zur erleichterten Bedienung ist vorzugsweise an der Abdeckung zumindest ein Griffelement zum Bewegen, insbesondere zum Verschieben, der Abdeckung relativ zur Öffnung vorgesehen. Das zumindest eine Griffelement umfasst zum Beispiel eine oder mehrere flügelartige Griffleisten. Das zumindest eine Griffelement ragt insbesondere über das Gehäuse.

Vorzugsweise weist das Gehäuse eine Frontfläche auf, in der die Öffnung vorgesehen ist, wobei die Frontfläche eine Krümmung umfasst, die einen ersten, vorzugsweise völlig planen, Frontflächen-Abschnitt und einen zweiten, vorzugsweise völlig planen, Frontflächen-Abschnitt verbindet, wobei der erste Frontflächen-Abschnitt und der zweite Frontflächen-Abschnitt gewinkelt (mit einem Winkel > 0°) zueinander angeordnet sind. Des Weiteren weist die Abdeckung eine der Frontfläche entsprechende Form mit einem ersten, vorzugsweise völlig planen, Abdeckungs-Abschnitt, einem zweiten, vorzugsweise völlig planen, Abdeckungs-Abschnitt und einer den ersten Abdeckungs-Abschnitt und den zweiten Abdeckungs-Abschnitt verbindenden Biegung auf. Vorzugsweise sind der erste Frontflächen-Abschnitt und der erste Abdeckungs-Abschnitt als schiefe Ebenen (in Bezug auf eine Grundfläche des Gehäuses der Reinigungs- und / oder Pflegevorrichtung und / oder auf den zweiten Frontflächen-Abschnitt und / oder auf den zweiten Abdeckungs-Abschnitt) ausgebildet. Damit ist die Bedienbarkeit der Reinigungs- und / oder Pflegevorrichtung, insbesondere das Beladen und / oder Entnehmen des Instruments aus den mehreren Kammern, erheblich erleichtert.

Vorzugsweise umfasst jede der mehreren Kammern zumindest eine Auslassöffnung zum Entfernen von Reinigungs- und / oder Pflegemittel aus der Kammer. Vorzugsweise ist die zumindest eine Auslassöffnung jeweils im Boden einer Kammer und / oder an dem zumindest einen Wandelement angeordnet. Vorzugsweise umfasst die zumindest eine Auslassöffnung an dem zumindest einen Wandelement einen oder mehrere Schlitze, die in dem Wandelement, zum Beispiel in der der Gehäuseöffnung zugewandten Schmalseite des Wandelements oder in dessen Seitenwand, angeordnet sind. Vorzugsweise ist die Auslassöffnung mit einer Absaugvorrichtung verbunden, welche ein in einer Kammer enthaltenes Reinigungs- und / oder Pflegemittel durch die Auslassöffnung absaugt. Damit kann in vorteilhafter Weise zum Beispiel überschüssiges Reinigungs- und / oder Pflegemittel, das aus einem zu reinigenden und / oder zu pflegenden Instrument ausgetreten ist, aus der Kammer entfernt werden.

Gemäß einem bevorzugten Ausführungsbeispiel umfasst die medizinische oder dentale Reinigungs- und / oder Pflegevorrichtung eine Detektoreinheit, die ausgebildet ist, zumindest eine Position der Abdeckung zu erkennen, insbesondere eine Abdeck- oder Verschlussposition, und ein der zumindest einen erkannten Position zugeordnetes oder zuordenbares Detektorsignal zu erzeugen. Die Detektoreinheit ist insbesondere ausgebildet, zumindest eine Position der Abdeckung in der Öffnung, relativ zur Öffnung und / oder relativ zu zumindest einer der mehreren Kammern zu erkennen. Besonders bevorzugt ist die Detektoreinheit ausgebildet, zu erkennen, dass zumindest eine der mehreren Kammer durch die Abdeckung vollständig verschlossen oder abgedeckt ist und / oder dass sich die Abdeckung in einer Abdeck- oder Verschlussposition befindet. Alternativ oder zusätzlich ist die Detektoreinheit ausgebildet, zu erkennen, dass zumindest eine der mehreren Kammern durch die Abdeckung nicht vollständig verschlossen oder abgedeckt ist.

Die Detektoreinheit ermöglicht damit in vorteilhafter Weise eine Vereinfachung und / oder Automatisierung des Betriebs der Reinigungs- und / oder Pflegevorrichtung und erhöht deren Betriebssicherheit. Die Detektoreinheit ermöglicht zum Beispiel aufgrund der Detektion, welche der mehreren Kammern durch die Abdeckung verschlossen (oder nicht verschlossen) ist, eine automatisierte Versorgung der zumindest einen verschlossenen Kammer und / oder des zumindest einen darin angeordneten Abgabeelementes mit einem Reinigungs- und / oder Pflegemittel. In entsprechender Weise ist auch ein automatisierter Start eines Reinigungs- und / oder Pflegeprozesses eines Instruments möglich, sobald eine der mehreren Kammern vollständig geschlossen ist (und ggf. zusätzlich eine vorbestimmte Zeitdauer vergangen ist). Entsprechend ist eine sofortige, automatisierte Unterbrechung der Förderung eines Reinigungs- und / oder Pflegemittel während eines Reinigungs- und / oder Pflegeprozesses möglich, wenn die Abdeckung von einer vollständig verschlossenen Kammer, in der ein Reinigungs- und / oder Pflegeprozesses abläuft, entfernt wird.

Vorzugsweise umfasst die Detektoreinheit eine magnetische Detektoreinheit, zum Beispiel ein Magnetelement und einen Magnet-Sensor, insbesondere einen Hall-Sensor. Alternativ kann die Detektoreinheit jedoch auch beliebige andere Detektoren umfassen und zum Beispiel als induktive, kapazitive oder optische Detektoreinheit ausgebildet sein.

Vorzugsweise ist zumindest ein Teil der Detektoreinheit, zum Beispiel zumindest ein Magnetelement, an oder in der Abdeckung vorgesehen und gemeinsam mit der Abdeckung bewegbar, insbesondere verschiebbar. Vorzugsweise ist zumindest ein Teil der Detektoreinheit, zum Beispiel zumindest ein Magnet-Sensor oder zumindest eine Spule, relativ zu der Abdeckung stationär in oder an dem Gehäuse angeordnet. Vorzugsweise ist zumindest ein Teil der Detektoreinheit, insbesondere das relativ zu der Abdeckung stationär angeordnete Teil, mehrfach vorgesehen. Vorzugsweise ist jeder der mehreren Kammern ein stationär angeordnetes Teil, zum Beispiel ein Magnet- oder Hall-Sensor, zugeordnet.

Vorzugsweise ist die Detektoreinheit derart ausgebildet, dass das gemeinsam mit der Abdeckung bewegbare Teil der Detektoreinheit und das stationär angeordnete Teil der Detektoreinheit dann am nächsten zueinander positioniert sind und / oder ein Detektorsignal erzeugen und / oder ein besonders starkes Detektorsignal erzeugen, wenn zumindest eine der mehreren Kammern vollständig durch die Abdeckung verschlossen ist und / oder mit Reinigungs- und / oder Pflegemittel versorgbar ist oder versorgt werden soll und / oder wenn eine Seitenkante der Abdeckung und ein Rand des Gehäuses oder die Gehäusekante einander kontaktieren, um die Bewegung, insbesondere das Verschieben, der Abdeckung zu begrenzen und / oder wenn die Abdeckung eine im Vorstehenden beschriebene Abdeck- oder Verschlussposition einnimmt.

Vorzugsweise umfasst die medizinische oder dentale Reinigungs- und / oder Pflegevorrichtung eine operativ mit der Detektoreinheit verbundene Steuer- oder Regelvorrichtung, die ausgebildet ist, das der zumindest einen erkannten Position zugeordnete oder zuordenbare Detektorsignal zu empfangen und zu verarbeiten und auf Basis des empfangenen und verarbeiteten Detektorsignals die Versorgung zumindest eines Abgabeelements und / oder zumindest einer Kammer mit zumindest einem Reinigungs- und / oder Pflegemittel zu steuern und / oder zu regeln. Insbesondere ist die Steuer- oder Regelvorrichtung ausgebildet, zumindest ein Abgabeelement zumindest einer der mehreren Kammern mit zumindest einem Reinigungs- und / oder Pflegemittel zu versorgen, wenn die Steuer- oder Regelvorrichtung auf Basis des empfangenen und verarbeiteten Detektorsignals erkennt, dass diese zumindest eine Kammer durch die Abdeckung (vollständig) verschlossen ist. Alternativ oder zusätzlich ist die Steuer- oder Regelvorrichtung ausgebildet, die Versorgung des zumindest einen Abgabeelements zumindest einer Kammer der mehreren Kammern mit zumindest einem Reinigungs- und / oder Pflegemittel zu unterbrechen, wenn die Steuer- oder Regelvorrichtung auf Basis des empfangenen und verarbeiteten Detektorsignals erkennt, dass diese zumindest eine Kammer durch die Abdeckung nicht (vollständig) verschlossen ist.

Vorzugsweise ist die Steuer- oder Regelvorrichtung ausgebildet, zumindest eines der im Vorstehenden genannten Steuerelemente der Versorgungsvorrichtung, zum Beispiel zumindest ein Ventil, eine Drossel oder eine Pumpe, zu steuern oder zu regeln, um die Versorgung zumindest eines Abgabeelements und / oder zumindest einer Kammer mit zumindest einem Reinigungs- und / oder Pflegemittel zu bewirken oder zu unterbrechen.

Ein erstes Verfahren zum Betrieb einer medizinischen oder dentalen Reinigungs- und / oder Pflegevorrichtung zur Reinigung und / oder Pflege zumindest eines medizinischen oder dentalen Instruments ist dadurch definiert, dass eine Abdeckung bewegt, insbesondere verschoben, wird, um einen Teil einer Öffnung in einem Gehäuse der Reinigungs- und / oder Pflegevorrichtung zu verschließen, durch welche mehrere voneinander getrennte Kammern mit medizinischen oder dentalen Instrumenten beladen werden können, so dass, wenn zumindest eine Kammer der mehreren Kammern vollständig verschlossen ist, zumindest eine andere Kammer der mehreren Kammern nicht vollständig verschlossen ist und insbesondere durch die Öffnung mit einem medizinischen oder dentalen Instrument beladen werden kann. Die medizinische oder dentale Reinigungs- und / oder Pflegevorrichtung umfasst insbesondere eine im Vorstehenden beschriebene Reinigungs- und / oder Pflegevorrichtung.

Ein zweites Verfahren zum Betrieb einer medizinischen oder dentalen Reinigungs- und / oder Pflegevorrichtung ist dadurch definiert, dass eine Detektoreinheit zumindest eine Position einer bewegbaren, insbesondere verschiebbaren, Abdeckung, die ausgebildet ist, einen Teil einer Öffnung in einem Gehäuse der Reinigungs- und / oder Pflegevorrichtung zu verschließen, durch welche mehrere voneinander getrennte Kammern mit medizinischen oder dentalen Instrumenten beladen werden können, erkennt und ein der zumindest einen erkannten Position zugeordnetes oder zuordenbares Detektorsignal erzeugt. Die medizinische oder dentale Reinigungs- und / oder Pflegevorrichtung umfasst insbesondere eine im Vorstehenden beschriebene Reinigungs- und / oder Pflegevorrichtung.

Besonders bevorzugt sind das erste und zweite Verfahren zum Betrieb einer medizinischen oder dentalen Reinigungs- und / oder Pflegevorrichtung miteinander verbindbar oder kombinierbar.

Vorzugsweise empfängt und verarbeitet die operativ mit der Detektoreinheit verbundene Steuer- oder Regelvorrichtung das der zumindest einen erkannten Position zugeordnete oder zuordenbare Detektorsignal und steuert und / oder regelt auf Basis des empfangenen und verarbeiteten Detektorsignals die Versorgung zumindest eines Abgabeelements und / oder zumindest einer der mehreren Kammern mit zumindest einem Reinigungs- und / oder Pflegemittel. Insbesondere steuert oder regelt die Steuer- oder Regelvorrichtung zumindest eines der im Vorstehenden genannten Steuerelemente der Versorgungsvorrichtung, zum Beispiel zumindest ein Ventil, eine Drossel oder eine Pumpe, um die Versorgung zumindest eines Abgabeelements und / oder zumindest einer Kammer mit zumindest einem Reinigungs- und / oder Pflegemittel zu bewirken oder zu unterbrechen.

Vorzugsweise werden die Abgabeelemente und / oder mehreren Kammern sequentiell und / oder alternierend mit zumindest einem Reinigungs- und / oder Pflegemittel versorgt. Besonders bevorzugt wird eines der Abgabeelemente und / oder eine der mehreren Kammern mit einem Reinigungs- und / oder Pflegemittel versorgt, während ein anderes Abgabeelement und / oder eine andere Kammer nicht mit einem Reinigungs- und / oder Pflegemittel versorgt wird. Besonders bevorzugt wird eines der Abgabeelemente und / oder eine der mehreren Kammern mit einem Reinigungs- und / oder Pflegemittel versorgt, während ein anderes Abgabeelement und / oder eine andere Kammer mit einem zu reinigenden und / oder zu pflegendem Instrument beladen wird oder ein gereinigtes und / oder gepflegtes Instrument von einem anderen Abgabeelement gelöst und / oder aus einer anderen Kammer entnommen wird.

Die Erfindung wird nachfolgend anhand bevorzugter Ausführungsbeispiele und Bezug nehmend auf die beigefügten Zeichnungen erläutert. Es zeigt die
Figur 1 eine medizinische oder dentale Reinigungs- und / oder Pflegevorrichtung zur Reinigung und / oder Pflege zumindest eines medizinischen oder dentalen Instruments, bei der eine verschiebbare Abdeckung sich in einer ersten Abdeck- oder Verschlussposition befindet, in der sie einen Teil einer Öffnung in einem Gehäuse der Reinigungs- und / oder Pflegevorrichtung derart verschließt, dass eine erste Kammer zur Aufnahme eines zu reinigenden und / oder zu pflegenden Instruments vollständig verschlossen und eine zweite Kammer vollständig geöffnet ist;
Figur 2 die medizinische oder dentale Reinigungs- und / oder Pflegevorrichtung der Figur 1, wobei die verschiebbare Abdeckung in einer Verschiebe- oder Zwischenposition angeordnet ist, in der keine der mehreren Kammern vollständig durch die Abdeckung verschlossen ist;
Figur 3 die medizinische oder dentale Reinigungs- und / oder Pflegevorrichtung der Figur 1, wobei die verschiebbare Abdeckung sich in einer zweiten Abdeck- oder Verschlussposition befindet, in welcher die erste Kammer vollständig geöffnet und die zweite Kammer vollständig verschlossen ist;
Figur 4 den in der Figur 3 mit "A" gekennzeichneten Ausschnitt, in dem ein Ausführungsbeispiel einer Detektoreinheit zu sehen ist, die ausgebildet ist, zumindest eine Position der Abdeckung zu erkennen und ein der zumindest einen erkannten Position zugeordnetes oder zuordenbares Detektorsignal zu erzeugen.

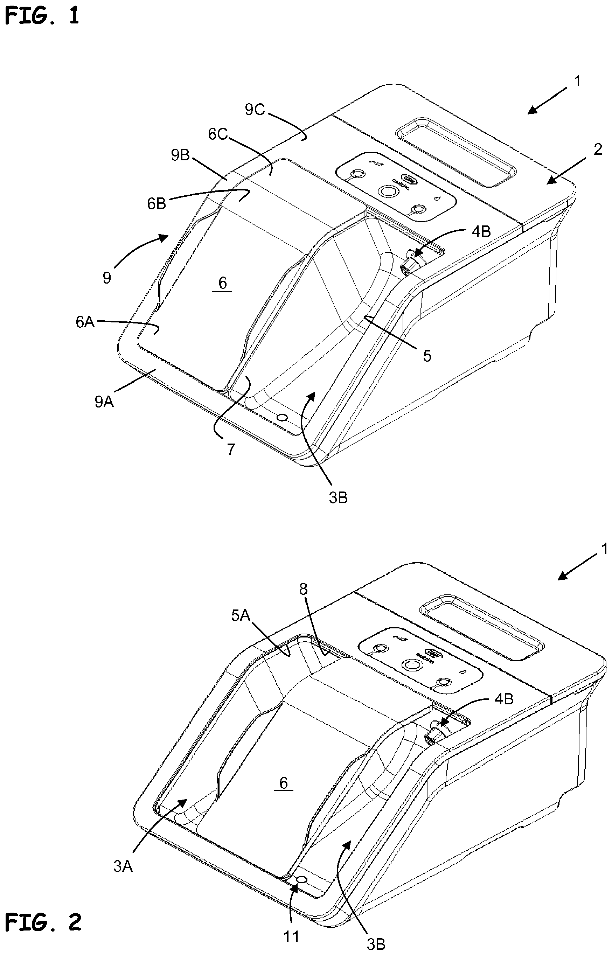

Die in den Figuren 1 - 3 dargestellte medizinische oder dentale Reinigungs- und / oder Pflegevorrichtung 1 zur Reinigung und / oder Pflege zumindest eines medizinischen oder dentalen Instruments, umfasst ein Gehäuse 2 und mehrere Kammern 3A, 3B zur Aufnahme von zumindest jeweils einem zu reinigenden und / oder zu pflegenden medizinischen oder dentalen Instrument.

Das Gehäuse 2 umfasst vorzugsweise eine Grundfläche oder Bodenplatte, eine Frontfläche 9 sowie mehrere zwischen der Grundfläche und der Frontfläche 9 angeordnete Seitenflächen oder Seitenwände. An einer rückwärtigen Seitenfläche ist vorzugsweise zumindest ein (lösbarer) Anschluss an eine Energiequelle und / oder an ein Fluidquelle vorgesehen. Das Gehäuse 2 ist vorzugsweise aus Kunststoff gefertigt.

Die mehreren Kammern 3A, 3B umfassen bei der in den Figuren 1 - 3 dargestellten medizinischen oder dentalen Reinigungs- und / oder Pflegevorrichtung 1 zwei Kammern 3A, 3B, wie im Vorstehenden bereits beschrieben, können jedoch auch mehr als zwei Kammern vorgesehen sein. Jede der Kammern 3A, 3B ist von länglicher, insbesondere rechteckiger, Form und weist einen Kammerboden sowie mehrere Seitenwände auf. Vorzugsweise ist der Kammerboden geneigt zur Grundfläche des Gehäuses 2, insbesondere in Richtung einer Auslassöffnung 11 zum Entfernen von (überschüssigem) Reinigungs- und / oder Pflegemittel aus den Kammern 3A, 3B, angeordnet. Besonders bevorzugt ist der Kammerboden von einem Abgabeelement 4A, 4B der Kammern 3A, 3B in Richtung der Auslassöffnung 11 geneigt.

Die Kammern 3A, 3B sind durch ein Wandelement 7 voneinander getrennt, wobei das Wandelement 7 vorzugsweise eine der mehreren Seitenwände der Kammern 3A, 3B bildet. Jede der Kammern 3A, 3B ist insbesondere derart bemessen, dass zumindest ein medizinisches oder dentales Instrument, vorzugsweise ein dentales Hand- oder Winkelstück, in ihr aufnehmbar ist.

In jeder Kammer 3A, 3B ist zumindest ein Abgabeelement 4A, 4B zur Abgabe zumindest eines Reinigungs- und / oder Pflegemittels in die jeweilige Kammer 3A, 3B und / oder an das zumindest eine, in der jeweiligen Kammer 3A, 3B aufgenommene, zu reinigende und / oder zu pflegende medizinische oder dentale Instrument vorgesehen. Das zumindest eine Abgabeelement 4A, 4B ist an einer der mehreren Seitenwände der Kammern 3A, 3B angeordnet und ragt in die jeweilige Kammer 3A, 3B. Das zumindest eine Abgabeelement 4A, 4B ist insbesondere als Stutzen, Fortsatz oder Kupplungselement mit zumindest einer Bohrung oder zumindest einem Kanal zum Leiten zumindest eines Reinigungs- und / oder Pflegemittels ausgebildet. Diese Bohrung oder dieser Kanal endet in einer Öffnung an der Oberfläche des Abgabeelements 4A, 4B, über die das zumindest eine Reinigungs- und / oder Pflegemittel an ein medizinisches oder dentales Instrument, das an das Abgabeelement 4A, 4B angeschlossen ist, abgebbar ist. Insbesondere ist das Reinigungs- und / oder Pflegemittel über die Öffnungen der Abgabeelemente 4A, 4B an einen Innenraum des Instruments und / oder ein darin angeordnetes Bauteil, zum Beispiel eine Leitung, ein Kanal, ein Rohr, ein Lagerelement oder ein Drehteil abgebbar.

Das zumindest eine Abgabeelement 4A, 4B ist mit einer (nicht dargestellten) Versorgungsvorrichtung verbunden, die ausgebildet ist, das zumindest eine Abgabeelement 4A, 4B mit zumindest einem Reinigungs- und / oder Pflegemittel zu versorgen, so wie dies im Vorstehenden schon detailliert beschrieben ist. Vorzugsweise verbindet die Versorgungsvorrichtung die Abgabeelemente 4A, 4B mit Quellen für die Reinigungs- und / oder Pflegemittel, zum Beispiel mit in oder an der Reinigungs- und / oder Pflegevorrichtung 1 angeordneten Behältern für die Reinigungs- und / oder Pflegemittel.

In dem Gehäuse 2 ist eine Öffnung 5 vorgesehen, durch welche die mehreren Kammern 3A, 3B mit medizinischen oder dentalen Instrumenten beladen werden können. Die Öffnung 5 erstreckt sich über alle der mehreren Kammern 3A, 3B, so dass alle Kammern 3A, 3B durch die Öffnung 5 zugänglich sind.

Des Weiteren ist eine Abdeckung 6 zum Verschließen der Öffnung 5 und der mehreren Kammern 3A, 3B, insbesondere während eines Reinigungs- und / oder Pflegevorgangs, vorgesehen. Wie aus den Figuren 1 - 3 zu erkennen ist, ist durch die Abdeckung 6 nur ein Teil der Öffnung 5 verschließbar oder abdeckbar, so dass, wenn zumindest eine Kammer 3A, 3B der mehreren Kammern 3A, 3B vollständig verschlossen oder abgedeckt ist, zumindest eine andere Kammer 3A, 3B der mehreren Kammern 3A, 3B nicht (vollständig) verschlossen oder abgedeckt ist und / oder durch die Öffnung 5 mit einem medizinischen oder dentalen Instrument beladbar ist.

Die Abdeckung 6 ist verschiebbar in der Öffnung 5 angeordnet, insbesondere entlang oder parallel zu einem Rand 5A der Öffnung 5. An diesem Rand 5A der Öffnung oder des Gehäuses 2 ist ein Führungselement 8 in Form einer Leiste vorgesehen, die in eine Nut an der Abdeckung 6 eingreift und die Abdeckung 6 stützt und führt. Der Rand 5A begrenzt auch die Bewegung der Abdeckung 6, in dem er einen Anschlag für die Abdeckung 6 bildet.

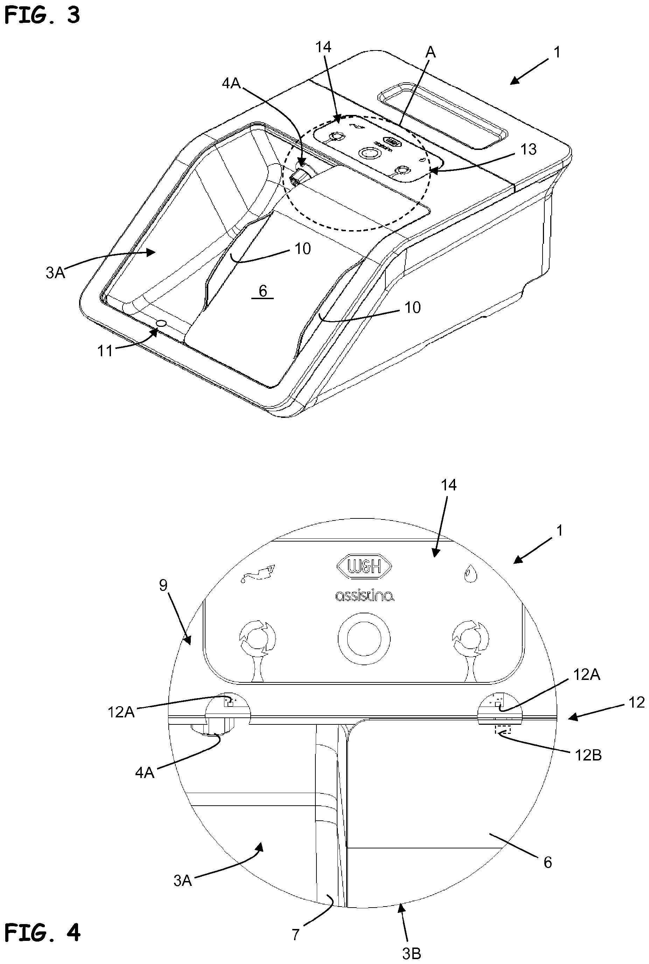

Die Verschiebbarkeit der Abdeckung 6 ist durch die unterschiedlichen Positionen, welche die Abdeckung 6 in den Figuren 1 - 3 einnimmt, dargestellt. In der Figur 1 befindet sich die Abdeckung 6 in einer Abdeck- oder Verschlussposition, in der die Abdeckung 6 die Kammer 3A vollständig verschließt oder abdeckt, insbesondere damit ein darin aufgenommenes Instrument gereinigt und / oder zu gepflegt werden kann, während die Kammer 3B geöffnet oder nicht verschlossen ist und somit insbesondere mit einem Instrument beladbar ist bzw. ein Instrument daraus entnehmbar ist. In der Figur 2 befindet sich die Abdeckung 6 in einer Verschiebe- oder Zwischenposition, in der sie keine der mehreren Kammern 3A, 3B vollständig verschließt oder abdeckt. Diese Verschiebe- oder Zwischenposition nimmt die Abdeckung 6 somit insbesondere beim Verschieben von einer Abdeck- oder Verschlussposition, beispielsweise an der Kammer 3A, in eine andere Abdeck- oder Verschlussposition, beispielsweise an der Kammer 3B, ein. Diese Abdeck- oder Verschlussposition an der Kammer 3B ist in der Figur 3 zu sehen. Die Abdeckung 6 verschließt oder deckt die Kammer 3B hierbei vollständig ab, insbesondere damit ein darin aufgenommenes Instrument gereinigt und / oder zu gepflegt werden kann, während die Kammer 3A geöffnet oder nicht verschlossen ist und somit insbesondere mit einem Instrument beladbar ist bzw. ein Instrument daraus entnehmbar ist.

An der Abdeckung 6 ist des Weiteren zumindest ein Griffelement 10 zum Verschieben der Abdeckung 6 relativ zu der Öffnung 5 vorgesehen ist. Das Griffelement 10 umfasst zwei Leisten oder Flügel, die von der Abdeckung 6 abstehen und die insbesondere an den seitlichen Enden der Abdeckung 6 angeordnet sind.

Die Frontfläche 9 des Gehäuses 2, in der die Öffnung 5 vorgesehen ist, weist eine Krümmung 9B auf, die einen ersten planen Frontflächen-Abschnitt 9A und einen zweiten planen Frontflächen-Abschnitt 9C verbindet, so dass der erste Frontflächen-Abschnitt 9A und der zweite Frontflächen-Abschnitt 9C gewinkelt zueinander angeordnet sind. Der Winkel beträgt maximal 90°, vorzugsweise jedoch merklich weniger, zum Beispiel weniger als 75°, weniger als 60° oder weniger als 55°. Die Abdeckung 6 weist eine der Frontfläche 9 entsprechende Form mit einem ersten planen Abdeckungs-Abschnitt 6A, einem zweiten planen Abdeckungs-Abschnitt 6C und einer den ersten Abdeckungs-Abschnitt 6A und den zweiten Abdeckungs-Abschnitt 6C verbindenden Biegung 6B auf.

In jeder der mehreren Kammern 3A, 3B ist zumindest eine Auslassöffnung 11 zum Entfernen von während eines Reinigungs- und / oder Pflegevorgangs in die Kammern 3A, 3B gelangtem, verbrauchtem oder überschüssigem Reinigungs- und / oder Pflegemittel aus der Kammer 3A, 3B vorgesehen. Die Auslassöffnung 11 ist insbesondere an einem tief oder nahe zur Grundfläche des Gehäuses 2 angeordneten Bereich des geneigten Kammerbodens vorgesehen. Das Reinigungs- und / oder Pflegemittel wird zum Beispiel über eine in der Reinigungs- und / oder Pflegevorrichtung 1 vorgesehene Absaugvorrichtung aus den Kammern 3A, 3B entfernt.

Die Figur 4 zeigt das mit "A" in der Figur 3 gekennzeichnete Detail der medizinischen oder dentalen Reinigungs- und / oder Pflegevorrichtung 1, wobei kleine Bereiche der Frontfläche 9 entfernt wurden, um einen Blick auf Teile einer im Gehäuse 2 angeordneten Detektoreinheit 12 freizugeben. Die Detektoreinheit 12 ist ausgebildet, zumindest eine Position der Abdeckung 6 zu erkennen, insbesondere zumindest eine Abdeck- oder Verschlussposition, und ein der zumindest einen erkannten Position zugeordnetes oder zuordenbares Detektorsignal zu erzeugen und / oder abzugeben.

Die Detektoreinheit 12 umfasst zumindest ein Teil 12B an der Abdeckung 6, das gemeinsam mit der Abdeckung 6 verschiebbar ist. Das Teil 12B ist zum Beispiel als Magnetelement ausgebildet und / oder insbesondere im Inneren der Abdeckung 6 angeordnet, zum Beispiel darin eingespritzt.

Die Detektoreinheit 12 umfasst des Weiteren zumindest ein Teil 12A, das relativ zu der Abdeckung 6 stationär in oder an dem Gehäuse 2 angeordnet ist. Wie aus der Figur 4 zu erkennen ist, ist jeder der mehreren Kammern 3A, 3B ein stationär angeordnetes Teil 12A, zum Beispiel ein Magnet-Sensor, zugeordnet. Befindet sich die Abdeckung 6 in einer Abdeck- oder Verschlussposition, in der zum Beispiel gemäß der Figur 4 die Kammer 3B vollständig von der Abdeckung 6 verschlossen ist, dann ist das Teil 12B, das zum Beispiel ein Magnetelement aufweist, dem Teil 12A, das zum Beispiel einen Magnet- oder Hallsensor aufweist, derart nahe, dass eine Detektion des Teils 12B durch das Teil 12A erfolgt und ein dieser Abdeck- oder Verschlussposition zugeordnetes oder zuordenbares Detektorsignal generiert und / oder abgegeben wird. Entsprechend ist die Detektoreinheit 12 ausgebildet, ein der Abdeck- oder Verschlussposition zugeordnetes oder zuordenbares Detektorsignal zu generieren und / oder abzugeben, wenn die Abdeckung 6 in einer der Figur 1 entsprechenden Abdeck- oder Verschlussposition die Kammer 3A vollständig verschließt. In der Verschiebe- oder Zwischenposition gemäß der Figur 2 detektiert das Teil 12A das Teil 12B nicht, zum Beispiel wegen eines zu großen Abstands zwischen diesen beiden Teilen 12A, 12B, so dass kein Detektionssignal generiert wird.

Die medizinische oder dentale Reinigungs- und / oder Pflegevorrichtung 1 umfasst eine operativ mit der Detektoreinheit 12 verbundene, im Inneren des Gehäuses 2 angeordnete Steuer- oder Regelvorrichtung 13, die ausgebildet ist, das der zumindest einen erkannten Position zugeordnete oder zuordenbare Detektorsignal zu empfangen und zu verarbeiten und auf Basis des empfangenen und verarbeiteten Detektorsignals die Versorgung zumindest eines Abgabeelements 4A, 4B und / oder zumindest einer der mehreren Kammer 3A, 3B mit zumindest einem Reinigungs- und / oder Pflegemittel zu steuern und / oder zu regeln, so wie dies im Vorstehenden schon ausführlich beschrieben ist. Insbesondere ist die Steuer- oder Regelvorrichtung 13 ausgebildet, einem in einer (vollständig) verschlossenen Kammer 3A, 3B angeordneten Abgabeelement 4A, 4B zumindest ein Reinigungs- und / oder Pflegemittel zukommen zu lassen und / oder einem in einer nicht (vollständig) verschlossenen Kammer 3A, 3B angeordneten Abgabeelement 4A, 4B kein Reinigungs- und / oder Pflegemittel zukommen zu lassen.

Schließlich ist am Gehäuse 2 ein Bedien- und / oder Anzeigepanel 14 vorgesehen, das insbesondere mit der Steuer- oder Regelvorrichtung 13 operativ verbunden ist. Das Panel 14 ist zum Beispiel ausgebildet, zumindest einen Betriebsstatus, Betriebsparameter, eine Fehlfunktion oder eine Wartungsaufforderung anzuzeigen. Alternativ oder zusätzlich umfasst das Panel 14 zumindest ein Betätigungselement, über das ein Anwender zum Beispiel unterschiedliche Betriebsmodi der Reinigungs- und / oder Pflegevorrichtung 1 einstellen kann.

Die beschriebenen oder dargestellten Ausführungsbeispiele dienen insbesondere der Veranschaulichung der Erfindung. Die in einem Ausführungsbeispiel offenbarten Merkmale sind daher nicht auf dieses Ausführungsbeispiel beschränkt, sondern sind einzeln oder gemeinsam mit einem oder mehreren Merkmalen eines der anderen Ausführungsbeispiele kombinierbar.

## Patentansprüche

1. Medizinische oder dentale Reinigungs- und / oder Pflegevorrichtung (1) zur Reinigung und / oder Pflege zumindest eines medizinischen oder dentalen Instruments, umfassend: ein Gehäuse (2), mehrere Kammern (3A, 3B) zur Aufnahme von zumindest jeweils einem zu reinigenden und / oder zu pflegenden medizinischen oder dentalen Instrument, wobei jede Kammer (3A, 3B) zumindest ein Abgabeelement (4A, 4B) zur Abgabe zumindest eines Reinigungs- und / oder Pflegemittels in die jeweilige Kammer (3A, 3B) und / oder an das zumindest eine, in der jeweiligen Kammer (3A, 3B) aufgenommene, zu reinigende und / oder zu pflegende medizinische oder dentale Instrument aufweist, eine Öffnung (5) in dem Gehäuse (2), durch welche die mehreren Kammern (3A, 3B) mit medizinischen oder dentalen Instrumenten beladen werden können, **gekennzeichnet durch**
eine einzige Abdeckung (6) zum Verschließen der Öffnung (5) und der mehreren Kammern (3A, 3B), so dass jede der mehreren Kammern (3A, 3B) durch die einzige Abdeckung (6) verschließbar oder abdeckbar ist, wobei durch die einzige Abdeckung (6) nur ein Teil der Öffnung (5) verschließbar ist, so dass, wenn zumindest eine Kammer (3A, 3B) der mehreren Kammern (3A, 3B) vollständig verschlossen ist, zumindest eine andere Kammer (3A, 3B) der mehreren Kammern (3A, 3B) nicht vollständig verschlossen ist und insbesondere durch die Öffnung (5) mit einem medizinischen oder dentalen Instrument beladbar ist.

2. Medizinische oder dentale Reinigungs- und / oder Pflegevorrichtung (1) nach Anspruch 1, **dadurch gekennzeichnet, dass**
die mehreren Kammern (3A, 3B) durch jeweils zumindest ein Wandelement (7) voneinander getrennt sind.

3. Medizinische oder dentale Reinigungs- und / oder Pflegevorrichtung (1) nach Anspruch 1 oder 2, **dadurch gekennzeichnet, dass**
die Abdeckung (6) beweglich, insbesondere verschiebbar, in oder an der Öffnung (5) angeordnet ist und / oder dass die Abdeckung (6) in oder entlang der Öffnung (5) bewegbar, insbesondere verschiebbar, ist.

4. Medizinische oder dentale Reinigungs- und / oder Pflegevorrichtung (1) nach einem der vorstehenden Ansprüche, **dadurch gekennzeichnet, dass**
die Öffnung (5) durch einen Rand (5A) begrenzt ist, an dem zumindest ein Führungselement (8) zum Bewegen, insbesondere zum Verschieben, der Abdeckung (6) vorgesehen ist und / oder der die Bewegung, insbesondere das Verschieben, der Abdeckung (6) begrenzt.

5. Medizinische oder dentale Reinigungs- und / oder Pflegevorrichtung (1) nach einem der vorstehenden Ansprüche, **dadurch gekennzeichnet, dass**
an der Abdeckung (6) zumindest ein Griffelement (10) zum Bewegen, insbesondere zum Verschieben, der Abdeckung (6) relativ zur Öffnung (5) vorgesehen ist.

6. Medizinische oder dentale Reinigungs- und / oder Pflegevorrichtung (1) nach einem der vorstehenden Ansprüche, **dadurch gekennzeichnet, dass**
das Gehäuse (2) eine Frontfläche (9) aufweist, in der die Öffnung (5) vorgesehen ist, wobei die Frontfläche (9) eine Krümmung (9B) aufweist, die einen ersten Frontflächen-Abschnitt (9A) und einen zweiten Frontflächen-Abschnitt (9C) verbindet, wobei der erste Frontflächen-Abschnitt (9A) und der zweite Frontflächen-Abschnitt (9C) gewinkelt zueinander angeordnet sind, und wobei die Abdeckung (6) eine der Frontfläche (9) entsprechende Form mit einem ersten Abdeckungs-Abschnitt (6A), einem zweiten Abdeckungs-Abschnitt (6C) und einer den ersten Abdeckungs-Abschnitt (6A) und den zweiten Abdeckungs-Abschnitt (6C) verbindenden Biegung (6B) aufweist.

7. Medizinische oder dentale Reinigungs- und / oder Pflegevorrichtung (1) nach einem der vorstehenden Ansprüche, **gekennzeichnet durch**
zumindest eine Auslassöffnung (11) zum Entfernen von Reinigungs- und / oder Pflegemittel aus der Kammer (3A, 3B), wobei die zumindest eine Auslassöffnung in jeder der mehreren Kammern (3A, 3B) und / oder in dem zumindest einen Wandelement (7) vorgesehen ist.

8. Medizinische oder dentale Reinigungs- und / oder Pflegevorrichtung (1) nach einem der vorstehenden Ansprüche, **gekennzeichnet durch**
eine Detektoreinheit (12), die ausgebildet ist, zumindest eine Position der Abdeckung (6) zu erkennen und ein der zumindest einen erkannten Position zugeordnetes oder zuordenbares Detektorsignal zu erzeugen.

9. Medizinische oder dentale Reinigungs- und / oder Pflegevorrichtung (1) nach Anspruch 8, **dadurch gekennzeichnet, dass**
zumindest ein Teil (12B) der Detektoreinheit (12) an der Abdeckung (6) vorgesehen ist und gemeinsam mit der Abdeckung (6) bewegbar, insbesondere verschiebbar, ist und / oder dass zumindest ein Teil (12A) der Detektoreinheit (12) relativ zu der Abdeckung (6) stationär in oder an dem Gehäuse (2) angeordnet ist.

10. Medizinische oder dentale Reinigungs- und / oder Pflegevorrichtung (1) nach Anspruch 8 oder 9, **gekennzeichnet durch**
eine operativ mit der Detektoreinheit (12) verbundene Steuer- oder Regelvorrichtung (13), die ausgebildet ist, das der zumindest einen erkannten Position zugeordnete oder zuordenbare Detektorsignal zu empfangen und zu verarbeiten und auf Basis des empfangenen und verarbeiteten Detektorsignals die Versorgung zumindest eines Abgabeelements (4A, 4B) und / oder zumindest einer der mehreren Kammer (3A, 3B) mit zumindest einem Reinigungs- und / oder Pflegemittel zu steuern und / oder zu regeln.

11. Medizinische oder dentale Reinigungs- und / oder Pflegevorrichtung (1) nach Anspruch 10, **dadurch gekennzeichnet, dass**
die Steuer- oder Regelvorrichtung (13) ausgebildet ist, das zumindest eine Abgabeelement (4A, 4B) und / oder die zumindest eine der mehreren Kammern (3A, 3B) mit zumindest einem Reinigungs- und / oder Pflegemittel zu versorgen, wenn die Steuer- oder Regelvorrichtung (13) auf Basis des empfangenen und verarbeiteten Detektorsignals erkennt, dass diese zumindest eine Kammer (3A, 3B) durch die Abdeckung (6) verschlossen ist.

12. Medizinische oder dentale Reinigungs- und / oder Pflegevorrichtung (1) nach Anspruch 10 oder 11, **dadurch gekennzeichnet, dass**
die Steuer- oder Regelvorrichtung (13) ausgebildet ist, die Versorgung des zumindest einen Abgabeelements (4A, 4B) und / oder der zumindest einen der mehreren Kammern (3A, 3B) mit zumindest einem Reinigungs- und / oder Pflegemittel zu unterbrechen, wenn die Steuer- oder Regelvorrichtung (13) auf Basis des empfangenen und verarbeiteten Detektorsignals erkennt, dass diese zumindest eine Kammer (3A, 3B) durch die Abdeckung (6) nicht verschlossen ist.

13. Verfahren zum Betrieb einer medizinischen oder dentalen Reinigungs- und / oder Pflegevorrichtung (1) nach einem der Ansprüche 8 - 12, **dadurch gekennzeichnet, dass**
die Detektoreinheit (12) zumindest eine Position der Abdeckung (6) erkennt und ein der zumindest einen erkannten Position zugeordnetes oder zuordenbares Detektorsignal erzeugt.

14. Verfahren zum Betrieb einer medizinischen oder dentalen Reinigungs- und / oder Pflegevorrichtung (1) nach Anspruch 13, **dadurch gekennzeichnet, dass**
die operativ mit der Detektoreinheit (12) verbundene Steuer- oder Regelvorrichtung (13) das der zumindest einen erkannten Position zugeordnete oder zuordenbare Detektorsignal empfängt und verarbeitet und auf Basis des empfangenen und verarbeiteten Detektorsignals die Versorgung zumindest eines Abgabeelements (4A, 4B) und / oder zumindest einer der mehreren Kammern (3A, 3B) mit zumindest einem Reinigungs- und / oder Pflegemittel steuert und / oder regelt.

15. Verfahren zum Betrieb einer medizinischen oder dentalen Reinigungs- und / oder Pflegevorrichtung (1) nach Anspruch 13 oder 14, **dadurch gekennzeichnet, dass**
die Abgabeelemente (4A, 4B) und / oder die mehreren Kammern (3A, 3B) sequentiell und / oder alternierend mit zumindest einem Reinigungs- und / oder Pflegemittel versorgt werden.

## Claims

1. A medical or dental cleaning and/or maintenance device (1) for cleaning and/or care of at least one medical or dental instrument comprising: a housing (2), a plurality of chambers (3A, 3B), each for accommodating at least one medical or dental instrument to be cleaned and/or cared for, wherein each chamber (3A, 3B) comprises at least one dispensing element (4A, 4B) for dispensing at least one cleaning agent and/or care agent into the respective chamber (3A, 3B) and/or to the at least one medical or dental instrument that is accommodated in the respective chamber (3A, 3B) and is to be cleaned and/or cared for, an opening (5) in the housing (2) through which the plurality of chambers (3A, 3B) can be loaded with medical or dental instruments, **characterized by**
a single cover (6) for closing the opening (5) and the plurality of chambers (3A, 3B), so that each of the plurality of chambers (3A, 3B) can be closed or covered by the single cover (6), wherein only a part of the opening (5) can be closed by the single cover (6) so that when at least one chamber (3A, 3B) of the plurality of chambers (3A, 3B) is completely closed, at least one other chamber (3A, 3B) of the plurality of chambers (3A, 3B) is not completely closed and in particular can be loaded with a medical or dental instrument through the opening (5).

2. The medical or dental cleaning and/or maintenance device (1) according to claim 1, **characterized in that**
the plurality of chambers (3A, 3B) is separated by at least one wall element (7).

3. The medical or dental cleaning and/or maintenance device (1) according to claim 1 or 2, **characterized in that**
the cover (6) is arranged movably, in particular displaceably in or on the opening (5) and/or the cover (6) is movable, in particular displaceable in or along the opening (5).

4. The medical or dental cleaning and/or maintenance device (1) according to any one of the preceding claims, **characterized in that**
the opening (5) is delimited by a border (5A) on which at least one guide element (8) is provided for moving, in particular for displacing, the cover (6) and/or which delimits the movement, in particular the displacement of the cover (6).

5. The medical or dental cleaning and/or maintenance device (1) according to any one of the preceding claims, **characterized in that**
at least one handle element (10) for moving, in particular displacing, the cover (6) relative to the opening (5) is provided on the cover (6).

6. The medical or dental cleaning and/or maintenance device (1) according to any one of the preceding claims, **characterized in that**
the housing (2) comprises a front face (9) in which the opening (5) is provided, wherein the front face (9) has a curvature (9B) which connects a first front face section (9A) and a second front face section (9C), wherein the first front face section (9A) and the second front face section (9C) are arranged at an angle to one another and wherein the cover (6) has a shape corresponding to that of the front face (9) with a first covering section (6A), a second covering section (6C) and a bend (6B) which connects the first covering section (6A) and the second covering section (6C).

7. The medical or dental cleaning and/or maintenance device (1) according to any one of the preceding claims, **characterized by**
at least one outlet opening (11) for removing cleaning agent and/or care agent from the chamber (3A, 3B), wherein the at least one outlet opening is provided in each one of the plurality of chambers (3A, 3B) and/or in the at least one wall element (7).

8. The medical or dental cleaning and/or maintenance device (1) according to any one of the preceding claims, **characterized by**
a detector unit (12), which is configured to detect at least one position of the cover (6) and to generate a detector signal that is or can be associated with at least one detected position.

9. The medical or dental cleaning and/or maintenance device (1) according to claim 8, **characterized in that**
at least one part (12B) of the detector unit (12) is provided on the cover (6) and is jointly movable, in particular displaceable, with the cover (6), and/or at least one part (12A) of the detector unit (12) is arranged relative to the cover (6) in a stationary position in or on the housing (2).

10. The medical or dental cleaning and/or maintenance device (1) according to claim 8 or 9, **characterized by**
a control or regulating device (13) which is operatively connected to the detector unit (12) and is configured to receive and to process the detector signal that is or can be associated with the at least one detected position and to control and/or regulate the supply of at least one cleaning agent and/or care agent to the at least one dispensing element (4A, 4B) and/or at least one of the plurality of chambers (3A, 3B) on the basis of the received and processed detector signal.

11. The medical or dental cleaning and/or maintenance device (1) according to claim 10, **characterized in that**
the control or regulating device (13) is configured to supply at least one cleaning agent and/or care agent to the at least one dispensing element (4A, 4B) and/or the at least one of the plurality of chambers (3A, 3B) when the control or regulating device (13) detects on the basis of the received and processed detector signal that this at least one chamber (3A, 3B) is closed by the cover (6).

12. The medical or dental cleaning and/or maintenance device (1) according to claim 10 or 11, **characterized in that**
the control or regulating device (13) is configured to interrupt the supply of at least one cleaning agent and/or care agent to the at least one dispensing element (4A, 4B) and/or the at least one of the plurality of chambers (3A, 3B) when the control or regulating device (13) detects on the basis of the received and processed detector signal that this at least one chamber (3A, 3B) is not closed by the cover (6).

13. A method for operating a medical or dental cleaning and/or maintenance device (1) according to any one of claims 8 - 12, **characterized in that**
the detector unit (12) detects at least one position of the cover (6) and generates a detector signal that is or can be associated with the at least one detected position.

14. The method for operating a medical or dental cleaning and/or maintenance device (1) according to claim 13, **characterized in that**
the control or regulating device (13) which is operatively connected to the detector unit (12) receives and processes the detector signal that is or can be associated with at least one detected position and, on the basis of the received and processed detector signal, controls and/or regulates the supply of at least one cleaning agent and/or care agent to the at least one dispensing element (4A, 4B) and/or at least one of the plurality of chambers (3A, 3B).

15. The method for operating a medical or dental cleaning and/or maintenance device (1) according to claim 13 or 14, **characterized in that**
the dispensing elements (4A, 4B) and/or the plurality of chambers (3A, 3B) are supplied sequentially and/or in alternation with at least one cleaning agent and/or care agent.

## Revendications

1. Dispositif de nettoyage et/ou d'entretien médical ou dentaire (1) pour le nettoyage et/ou l'entretien d'au moins un instrument médical ou dentaire, comprenant: un boîtier (2), plusieurs compartiments (3A, 3B) pour réceptionner respectivement au moins un instrument médical ou dentaire à nettoyer et/ou à entretenir, dans lequel chaque compartiment (3A, 3B) présente au moins un élément d'émission (4A, 4B) pour l'émission d'au moins un produit de nettoyage et/ou d'entretien dans le compartiment (3A, 3B) respectif et/ou à l'au moins un instrument médical ou dentaire à nettoyer et/ou à entretenir qui est réceptionné dans le compartiment (3A, 3B) respectif, une ouverture (5) dans le boîtier (2) à travers laquelle les plusieurs compartiments (3A, 3B) peuvent être chargés avec des instruments médicaux ou dentaires, **caractérisé par**
un unique couvercle (6) pour la fermeture de l'ouverture (5) et des plusieurs compartiments (3A, 3B) de sorte que chacun des plusieurs compartiments (3A, 3B) peut être fermé ou recouvert par l'unique couvercle (6), dans lequel grâce à l'unique couvercle (6), seulement une partie de l'ouverture (5) peut être fermée de sorte que, lorsqu'au moins un compartiment (3A, 3B) des plusieurs compartiments (3A, 3B) est complètement fermé, au moins un autre compartiment (3A, 3B) des plusieurs compartiments (3A, 3B) n'est pas complètement fermé et peut en particulier être chargé à travers l'ouverture (5) avec un instrument médical ou dentaire.

2. Dispositif de nettoyage et/ou d'entretien médical ou dentaire (1) selon la revendication 1, **caractérisé en ce que**
les plusieurs compartiments (3A, 3B) sont séparés entre eux respectivement par au moins un élément de paroi (7).

3. Dispositif de nettoyage et/ou d'entretien médical ou dentaire (1) selon la revendication 1 ou 2, **caractérisé en ce que**
le couvercle (6) est disposé de manière mobile, en particulier coulissante, dans ou au niveau de l'ouverture (5) et/ou **en ce que** le couvercle (6) est mobile, en particulier coulissant, dans l'ouverture (5) ou le long de celle-ci.

4. Dispositif de nettoyage et/ou d'entretien médical ou dentaire (1) selon l'une des revendications précédentes, **caractérisé en ce que**
l'ouverture (5) est délimitée par un rebord (5A) au niveau duquel au moins un élément de guidage (8) est prévu pour faire bouger, en particulier coulisser le couvercle (6) et/ou lequel limite le mouvement, en particulier le coulissement du couvercle (6).

5. Dispositif de nettoyage et/ou d'entretien médical ou dentaire (1) selon l'une des revendications précédentes, **caractérisé en ce que**
l'on prévoit, au niveau du couvercle (6), au moins un élément de poignée (10) pour faire bouger, en particulier coulisser le couvercle (6) par rapport à l'ouverture (5).

6. Dispositif de nettoyage et/ou d'entretien médical ou dentaire (1) selon l'une des revendications précédentes, **caractérisé en ce que**
le boîtier (2) présente une surface frontale (9) dans laquelle l'ouverture (5) est prévue, dans lequel la surface frontale (9) présente une courbure (9B) qui relie une première partie de surface frontale (9A) et une deuxième partie de surface frontale (9C), dans lequel la première partie de surface frontale (9A) et la deuxième partie de surface frontale (9C) sont disposées en angle l'une par rapport à l'autre, et dans lequel le couvercle (6) présente une forme correspondant à la surface frontale (9) avec une première partie de couvercle (6A), une deuxième partie de couvercle (6C), et une courbure (6B) reliant la première partie de couvercle (6A) et la deuxième partie de couvercle (6C).

7. Dispositif de nettoyage et/ou d'entretien médical ou dentaire (1) selon l'une des revendications précédentes, **caractérisé par**
au moins une ouverture de sortie (11) pour l'élimination de produit de nettoyage et/ou d'entretien du compartiment (3A, 3B), dans lequel l'au moins une ouverture de sortie est prévue dans chacun des plusieurs compartiments (3A, 3B) et/ou dans l'au moins un élément de paroi (7).

8. Dispositif de nettoyage et/ou d'entretien médical ou dentaire (1) selon l'une des revendications précédentes, **caractérisé par**
une unité de détection (12), laquelle est réalisé pour reconnaître au moins une position du couvercle (6) et pour générer un signal de détection associé, ou pouvant être associé, à l'au moins une position reconnue.

9. Dispositif de nettoyage et/ou d'entretien médical ou dentaire (1) selon la revendication 8, **caractérisé en ce que**
au moins une partie (12B) de l'unité de détection (12) est prévue au niveau du couvercle (6) et peut bouger, en particulier coulisser, ensemble avec le couvercle (6) et/ou **en ce qu'**au moins une partie (12A) de l'unité de détection (12) est disposée de manière stationnaire par rapport au couvercle (6) dans ou au niveau du boîtier (2).

10. Dispositif de nettoyage et/ou d'entretien médical ou dentaire (1) selon la revendication 8 ou 9, **caractérisé par**
un dispositif de commande ou de régulation (13) relié de manière fonctionnelle à l'unité de détection (12), lequel est réalisé pour réceptionner et traiter le signal de détection associé, ou pouvant être associé, à l'au moins une position reconnue et, sur la base du signal de détection réceptionné et traité, pour commander et/ou réguler l'alimentation d'au moins un élément d'émission (4A, 4B) et/ou d'au moins l'un des plusieurs compartiments (3A, 3B) avec au moins un produit de nettoyage et/ou d'entretien.

11. Dispositif de nettoyage et/ou d'entretien médical ou dentaire (1) selon la revendication 10, **caractérisé en ce que**
le dispositif de commande ou de régulation (13) est réalisé pour alimenter l'au moins un élément d'émission (4A, 4B) et/ou l'au moins un des plusieurs compartiments (3A, 3B) avec au moins un produit de nettoyage et/ou d'entretien si le dispositif de commande ou de régulation (13) reconnaît, sur la base du signal de détection réceptionné et traité, que cet au moins un compartiment (3A, 3B) est fermé par le couvercle (6).

12. Dispositif de nettoyage et/ou d'entretien médical ou dentaire (1) selon la revendication 10 ou 11, **caractérisé en ce que**
le dispositif de commande ou de régulation (13) est réalisé pour interrompre l'alimentation de l'au moins un élément d'émission (4A, 4B) et/ou de l'au moins un des plusieurs compartiments (3A, 3B) avec au moins un produit de nettoyage et/ou d'entretien si le dispositif de commande ou de régulation (13) reconnaît, sur la base du signal de détection réceptionné et traité, que cet au moins un compartiment (3A, 3B) n'est pas fermé par le couvercle (6).

13. Procédé pour faire fonctionner un dispositif de nettoyage et/ou d'entretien médical ou dentaire (1) selon l'une des revendications 8 - 12, **caractérisé en ce que** l'unité de détection (12) reconnaît au moins une position du couvercle (6) et génère un signal de détection associé, ou pouvant être associé, à l'au moins une position reconnue.

14. Procédé pour faire fonctionner un dispositif de nettoyage et/ou d'entretien médical ou dentaire (1) selon la revendication 13, **caractérisé en ce que**
le dispositif de commande ou de régulation (13) relié de manière fonctionnelle à l'unité de détection (12) réceptionne et traite le signal de détection associé, ou pouvant être associé, à l'au moins une position reconnue et, sur la base du signal de détection réceptionné et traité, commande et/ou régule l'alimentation d'au moins un élément d'émission (4A, 4B) et/ou d'au moins l'un des plusieurs compartiments (3A, 3B) avec au moins un produit de nettoyage et/ou d'entretien.

15. Procédé pour faire fonctionner un dispositif de nettoyage et/ou d'entretien médical ou dentaire (1) selon la revendication 13 ou 14, **caractérisé en ce que** les éléments d'émission (4A, 4B) et/ou les plusieurs compartiments (3A, 3B) sont alimentés séquentiellement et/ou en alternance avec au moins un produit de nettoyage et/ou d'entretien.
